# EUROPÄISCHE PATENTSCHRIFT

(11) **EP 1 594 949 B1**
(45) Veröffentlichungstag und Bekanntmachung des Hinweises auf die Patenterteilung: **26.10.2011**
(21) Anmeldenummer: 03785710.9
(22) Anmeldetag: 02.12.2003
(51) Int. Cl.: C12M 3/00, C12M 1/26

(54) **VERFAHREN UND VORRICHTUNG ZUR BILDUNG VON BIOLOGISCHEM ZELLMATERIAL**
METHOD AND DEVICE FOR THE FORMATION OF BIOLOGICAL CELL MATERIAL
PROCEDE ET DISPOSITIF DE FORMATION D'UNE MATIERE CELLULAIRE BIOLOGIQUE

(30) Priorität: 21.02.2003 DE 10307487
(43) Veröffentlichungstag der Anmeldung: 16.11.2005
(73) Patentinhaber: Fraunhofer-Gesellschaft zur Förderung der angewandten Forschung e.V., 80686 München (DE)
(72) Erfinder: FUHR, Günter, R., 13187 Berlin (DE); ZIMMERMANN, Heiko, 66113 Saarbrücken (DE)
(74) Vertreter: Hertz, Oliver
(86) Internationale Anmeldenummer: PCT/EP2003/013580
(87) Internationale Veröffentlichungsnummer: WO 2004/074425

(56) Entgegenhaltungen:
- EP-A- 0 307 085
- US-A- 5 108 926
- US-A- 5 866 417
- US-A- 5 900 361

## Beschreibung

Die Erfindung betrifft Verfahren zur Bildung, insbesondere zur Formgebung und/oder Deposition von Zellmaterial mit einer Vielzahl biologischer Zellen, insbesondere Verfahren zu Einstellung oder Veränderung einer Oberflächentopographie eines Zellmaterials und Verfahren zur geometrischen Strukturierung von Zellmaterial, wie z. B. Verfahren zum sog. Tissue Engineering. Die Erfindung betrifft auch Manipulationswerkzeuge zur Durchführung dieser Verfahren, insbesondere Substrate für Zellmaterial wie z. B. Zellkulturen oder Gewebe und Formwerkzeuge, mit denen die geometrische Form und/oder Ausdehnung des Zellmaterials veränderbar sind. Die Erfindung betrifft auch neue Anwendungen der genannten Verfahren und Manipulationswerkzeuge.

In der Medizin, Biotechnologie und Biochemie bestehen wesentliche Aufgaben bei der Untersuchung oder Manipulation biologischer Zellen, insbesondere in Zusammenhang mit der medizinischen Zelltherapie und der Gewebebearbeitung (Tissue Engineering) darin, dass Zellformationen oder Zellgruppen mit einer vorbestimmten geometrischen Anordnung der einzelnen Zellen bereitgestellt werden. Beispielsweise soll die Form eines Zellmaterials, das in einen Organismus implantiert wird, möglichst gut an die geometrischen Bedingungen am Implantationsort angepasst sein. Aus der Praxis ist bekannt, die Form des Implantatmaterials durch eine passende mechanische Abtrennung (Abschneiden) von einer Zellkultur einzustellen. Dies ist jedoch nachteilig, da Schädigungen der Zellen oder des Zellmaterials unerwünschte Wirkungen bei der Geweberegeneration nach der Implantation haben können. Bei zahlreichen, in der Praxis bekannt gewordenen Experimenten trat nicht die gewünschte Regeneration oder Neubildung eines Zell- oder Gewebetyps ein, sondern bspw. eine Induktion von Tumoren. Es wird davon ausgegangen, dass die Induktion von Tumoren als unkontrollierte Zellvermehrung von Zellen durch physikalische, chemische oder mechanische Fremdeinflüsse zum Implantationsort befördert wird. Diese Einflüsse können mit den herkömmlichen Techniken nicht reproduzierbar eingestellt oder wenigstens erfasst werden.

Ein weiteres Beispiel für die Formung von Zellmaterial besteht bei der Untersuchung von Wirkstoffen (Prüfung von pharmakologischen Wirkstoffen) an Gewebemodellen. Als Gewebemodelle werden bevorzugt sog. Sphäroide verwendet, die als kugelförmige Gebilde durch schichtförmiges Aufwachsen von Zellmaterial auf einem inneren Kern aus Zellen hergestellt werden. Ein Nachteil herkömmlicher Gewebemodelle besteht in deren beschränkter Größe. Beispielsweise können Sphäroide bisher lediglich bis zu einem Durchmesser von rd. 150 um verwendet werden. Bei größeren Durchmessern treten Probleme bei der Nährstoffversorgung der inneren Zellen auf. Die Vitalität der inneren Zellen wird beschränkt und es kann zu einem Absterben der Zellen von innen nach außen kommen.

Ein weiteres, bisher ungelöstes Problem beim Tissue Engineering besteht in der Erzeugung von strukturierten Kompositmaterialien aus Zellmaterialien verschiedener Zellarten oder aus biologischen Zellen und synthetischen Materialien. Ein Nachteil besteht darin, dass bisher Kompositbildungen mit mechanischen Verletzungen von Zellen oder Zellmaterial verbunden sind.

Die genannten Probleme bei der Zelltherapie und die bisher zum Teil unbefriedigenden Ergebnisse beim Tissue Engineering stellen gegenwärtig die wichtigsten-Beschränkungen und Verzögerungen vor einer breiten Anwendung dieser Verfahren in der Biotechnologie und der Medizin dar.

US-A-5 108 926 offenbart eine gezielte Anordnung biologischer Zellen für eine Gewebekultur auf einem Substrat mit einem nach Art eines Tintenstrahldruckers arbeitenden Druckverfahren.

Die Aufgabe der Erfindung ist es, verbesserte Verfahren zur Bildung von Zellmaterial mit einer vorbestimmten geometrischen Anordnung der einzelnen Zellen bereitzustellen, mit denen die Nachteile herkömmlicher Verfahren überwunden werden und die insbesondere geeignet sind, Zellmaterial ohne eine Verletzung von Einzelzellen zu formen und/oder zu erzeugen. Die Aufgabe der Erfindung ist es auch, verbesserte Manipulationswerkzeuge zur Durchführung derartiger Verfahren bereitzustellen, mit denen die Nachteile herkömmlicher Zell- oder Gewebetechniken überwunden werden. Eine weitere Aufgabe der Erfindung ist es, neue Anwendungen der Formung, Kultivierung oder Erzeugung von Zellmaterial anzugeben.

Diese Aufgaben werden mit Verfahren und Manipulationswerkzeugen mit den Merkmalen gemäß den Patentansprüchen 1 oder 20 gelöst. Vorteilhafte Ausführungsformen und Anwendungen der Erfindung ergeben sich aus den abhängigen Ansprüchen.

Verfahrensbezogen basiert die Erfindung auf der allgemeinen technischen Lehre, mit einem Manipulationswerkzeug, das ein zu manipulierendes Zellmaterial zumindest teilweise berührt, die geometrische Anordnung von Zellen im Zellmaterial einzustellen, indem die Oberflächengeometrie des Manipulationswerkzeugs in vorbestimmter Weise geändert wird. Die Oberflächengeometrie wird erfindungsgemäß in Bezug auf mindestens eines der Merkmale Fläche, Ausrichtung relativ zu einem ortsfesten Bezugssystem, Oberflächenform und Oberflächenstruktur verändert Vorteilhafterweise folgen die Zellen des Zellmaterials, die die Werkzeugoberfläche berühren, der Oberflächenbewegung, wobei die Zellen bei der Oberflächenbewegung in ihrem physikalischen und chemischen Zustand unverändert bleiben. Die geometrische Veränderung der Werkzeugoberfläche bewirkt ausschließlich eine Umordnung der Zellen, insbesondere eine verletzungsfreie Verdrängung oder Verschiebung von Zellen, bei der zwar Zellen in Kontakt mit dem Werkzeugkörper oder tiefer im Zellmaterial liegende Zellen deformiert oder in ihrer räumlichen Lage verändert werden, dabei jedoch keine chemischen Signale in Form von Botenstoffen oder Substanzabsonderungen abgeben.

Unter Zellmaterial wird hier allgemein eine Anhäufung von Zellen verstanden, die über Adhäsionskontakte (makromolekulare chemische Verbindungen, keine van der Waals-Bindung) mit ihrer Umgebung in Verbindung stehen. Das Zellmaterial ist beispielsweise ein Verbund oder Zusammenschluss von Einzelzellen, ein Gewebe (Verbund gleichartig differenzierter Zellen) oder ein Organ. Der nicht-flüssige Verbund von Einzelzellen kann zusätzliche synthetische Komponenten, z. B. ein synthetisches Matrixmaterial enthalten. Mit Bildung von Zellmaterial wird hier allgemein eine Veränderung der Form, Dichte, Größe und/oder Struktur von Zellmaterial bezeichnet.

Das Manipulationswerkzeug ist allgemein ein Fremdkörper oder Objekt aus einem in Bezug auf das Zellmaterial abgrenzbaren Material mit einer festen Oberfläche, deren geometrischen Eigenschaften zumindest in Teilbereichen veränderlich ist. Das Manipulationswerkzeug kann zum Beispiel ein Substrat für eine Zellkultur, eine Sonde im Zellmaterial oder einen Stempel zur Formgebung aufweisen. Das Manipulationswerkzeug ist derart verstellbar, dass sich geometrische Eigenschaften von seiner Oberfläche in Bezug auf mindestens eines der oben genannten Merkmale verändern. Die Geschwindigkeit der Verstellung wird als Vortriebsgeschwindigkeit bezeichnet.

Die Erfindung basiert insbesondere auf den folgenden Überlegungen der Erfinder. Es wurde erstens erkannt, dass die bisher verschiedenartig ausfallenden Reaktionen bspw. von Zellen in einem Gewebe oder einen Zellverbund darin begründet liegen, dass durch die Einführung eines Werkzeugs Zellen im vorhandenen Zellmaterial verletzt oder zerstört und damit Wundeffekte hervorgerufen werden. Bei einer Zell- oder Gewebeverwundung werden chemische Signale (Aussendung molekularer Botenstoffe) oder zellulär getragene Prozesse, wie z. B. eine Fibroplasteneinwanderung, eine Fibronektinaussonderung oder dgl. erzeugt. Die Reaktion verletzter Zellen beeinflusst die Wirkung der Zellen oder Zusatzstoffe. Beispielsweise verhalten sich Stammzellen in der Umgebung einer Zellverwundung anders als Stammzellen in einem intakten Zellmaterial. Zweitens haben die Erfinder festgestellt, dass entgegen bisherigen Vorstellungen selbst adhäsiv gebundene Zellen verletzungsfrei räumlich verdrängt werden können. Dies ermöglicht die mechanische Einführung von Werkzeugen in Zellmaterial. Die Zellen bleiben bei der Bewegung der Werkzeugoberfläche in und/oder durch das Zellmaterial unverletzt, wenn die Vortriebsgeschwindigkeit genügend klein ist, dass sich die Adhäsionskontakte zwischen den Zellen auf natürliche, d. h. die Zellen nicht beeinflussende oder zerstörende Weise lösen und in der sich verändernden Umgebung neu bilden lassen.

Durch die geometrische Verstellung der Werkzeugoberfläche und die damit verbundene verletzungsfreie Verdrängung oder Verschiebung von Zellen können die o. g. Anforderungen vollständig erfüllt werden. Eine Schädigung oder Beeinträchtigung des Zellmaterials wird ausgeschlossen. Der physikalische, chemische und mechanische Zustand der Zellen kann vollständig charakterisiert werden und erhalten bleiben. Schädliche Kontakte zwischen Zellen und Oberflächen von Fremdkörpern werden vermieden, zelluläre Signale durch unerwünschte Oberflächenberührungen werden unterbunden. Durch die verletzungsfreie Bewegung erfolgt die Zellmanipulation ideal schonend. Das Manipulationswerkzeug kann zielgenau in eine bestimmte Konfiguration im Zellmaterial geführt werden.

Gemäß einer bevorzugten Ausführungsform der Erfindung wird das Manipulationswerkzeug mit einer Vortriebsgeschwindigkeit verstellt, die kleiner oder gleich einer Bezugsgeschwindigkeit ist, die durch die physiologische Bindungsrate biologischer Zellen bestimmt wird (Bindungsgeschwindigkeit der Zellen bei deren natürlicher Zellbewegung). Die natürliche Zellbewegung (englisch: cell locomotion) umfasst die Ortsänderung einer kompletten Zelle auf einer festen Oberfläche oder in Zellmaterial durch eine Umordnung von Adhäsionskontakten von Zellorganen (Membranorgane, zum Beispiel Membranausstülpungen), wie sie beispielsweise von M. Abercrombie et al. in der Publikation "The Locomotion Of Fibroblasts In Culture" ("Experimental Cell Research", Bd. 67, 1971, S. 359-367) und von L. P. Cramer in der Publikation "Organization and polarity of actin filament networks in cells: implications for the mechanism of myosin-based cell motility" ("Biochem. Soc. Symp." Bd. 65, 1999, S. 173-205) beschrieben wird.

Die physiologische Bezugsgeschwindigkeit ist an sich bekannt (siehe z. B. G. Fuhr et al. in "Biol. Chem.", 1998, Bd. 379, S. 1161-1173) oder an tierischen oder humanen Zellen messbar. Die interessierende Bindungsrate ist bspw. durch Messung der Dynamik von Adhäsionsmustern einzelner Zellen auf künstlichen Oberflächen ableitbar.

Mit der Vortriebsgeschwindigkeit wird hier allgemein die Geschwindigkeit bezeichnet, mit der sich die Oberfläche des Manipulationswerkzeugs relativ zum Zellmaterial bewegt. Diese Geschwindigkeit kann sich auf die gesamte Oberfläche an sich, bestimmte Formelemente, die Teile der Oberfläche repräsentieren, oder eine Oberflächenvergrößerung beziehen. Bei einer Oberflächendehnung bezeichnet die Vortriebsgeschwindigkeit zum Beispiel die relative Geschwindigkeit von Bezugspunkten bei einer Vergrößerung der Oberfläche. Bei Einstellung der Vortriebsgeschwindigkeit kann das Manipulationswerkzeug vorteilhafterweise verletzungsfrei Zellen in einem natürlich gegebenen Verbund bewegen oder umordnen. Die Vortriebsgeschwindigkeit wird an die permanent im Gewebe stattfindende Zellbewegung angepasst. Es ist bspw. bekannt, dass sich bestimmte Typen von Immunzellen (z. B. Makrophagen) durch Verdrängung vorhandener Zellen selbst durch dichtes Gewebe bewegen. Die Erfinder haben festgestellt, dass überraschenderweise diese Verdrängungsbewegung auch mit Werkzeugen, die erheblich größer als Immunzellen sind und makroskopische Dimensionen im Sub-Millimeter- bis Zentimeterbereich besitzen, bei Einstellung der genannten Vortriebsgeschwindigkeit realisierbar ist. Während der Bewegung oder Herstellung der Werkzeugoberfläche werden laufend makromolekulare Bindungen (zum Beispiel membranständige Makromoleküle der Integrin- und Catherinfamilie) zwischen den Zellen getrennt und zum Beispiel mit der Sondenoberfläche neu geknüpft.

Besondere Vorteile der Erfindung ergeben sich, wenn die Vortriebsgeschwindigkeit des Manipulationswerkzeugs in einem Geschwindigkeitsbereich von 0.1 um/h bis 1 mm/h, vorzugsweise im Bereich von 1 um/h bis zu 500 um/h gewählt wird. In diesem Geschwindigkeitsbereich liegen die Bindungsraten des Aufbaus und des Abbaus von makromolekularen Bindungen, die typischerweise durch meinbranständige Makromoleküle der Integrin- und Catherinfamilie vermittelt werden. Die bevorzugten Geschwindigkeitsbereiche entsprechen den Geschwindigkeiten der Zellbewegung von insbesondere Fibroplasten, Makrophagen, Lymphozyten, Chondrozyten oder Tumorzellen. Vorteilhafterweise kann bei Einstellung einer derart geringen Vortriebsgeschwindigkeit die Position und Form der Werkzeugoberfläche mit einer hohen Genauigkeit von rund +/-1 µm oder sogar darunter eingestellt werden. Die Vortriebsgeschwindigkeiten in den genannten Bereichen entsprechen den aktiven endogenen Bewegungsgeschwindigkeiten von Zellen in und auf Gewebe. Die Bewegung der Sonde bewirkt damit einen permanenten An- und Umbau der Zellen in der unmittelbaren Umgebung der Werkzeugoberfläche, wobei durch eine permanent wirkende Vortriebskraft eine Verdrängung der Zellen gefördert werden kann.

Wenn das Manipulationswerkzeug einer permanent wirkenden Vortriebskraft ausgesetzt wird, kann seine Bewegung mit der gewünschten Vortriebsgeschwindigkeit vorteilhafterweise selbst bei geringstem Kraftaufwand durchgeführt werden. Dies ermöglicht die Verwendung von Stelleinrichtungen mit geringer Leistung. Wenn die Vortriebskraft durch eine mechanische Druckkraft gebildet wird, können sich Vorteile für die Übertragung der Vortriebskraft auf die Sonde ergeben. Wenn die Vortriebskraft durch Kräfte in elektrischen oder magnetischen Feldern gebildet wird, können sich Vorteile ergeben, da die Vortriebskräfte über eine Fernwirkung ausgeübt werden können.

Besondere Vorteile können sich ergeben, wenn das erfindungsgemäße Verfahren an Zellmaterial ausgeführt wird, das sich außerhalb eines tierischen oder menschlichen Organismus befindet. Das Zellmaterial kann unter geeigneten Kultivierungsbedingungen auf einem festen Träger angeordnet werden, der die Gegenkraft zur Ausübung der Vortriebskraft aufbringt. Das Zellmaterial und die Sonde können mit hoher Präzision positioniert werden.

Alternativ kann sich das Zellmaterial im Verbund in einem lebenden Organismus befinden. Das Manipulationswerkzeug kann bspw. als Sonde, Biopsiewerkzeug, Injektionswerkzeug oder Implantationswerkzeug in Gewebe eingeführt werden. Die Einführung erfolgt wegen der geringen Vortriebsgeschwindigkeit in einem Zustand, in dem das betroffene Gewebe fixiert, z. B. mit dem umliegenden Teil des Organismus ortsfest auf einem Träger gehaltert ist. Die Verwendung eines Narkosemittels ist für die Ruhestellung bevorzugt, mit Blick auf die Verletzungsfreiheit des Verfahrens jedoch nicht zwingend erforderlich.

Gemäß einer weiteren bevorzugten Ausführungsform der Erfindung kann die Verstellung des Manipulationswerkzeugs eine Ausdehnung der Werkzeugoberfläche umfassen. Das zu manipulierende Zellmaterial wird auf der Werkzeugoberfläche angeordnet. Das Zellmaterial bildet eine Zellschicht, deren Dicke sich durch die Ausdehnung der Oberfläche vermindert und/oder in der Lücken entstehen, in denen die Werkzeugoberfläche freiliegt. Vorteilhafterweise wird damit die Bildung eines sich vergrößernden Schichtverbundes des Zellmaterials durch Anwachsen von Zellen aus einem angrenzenden Kultivierungsmedium ermöglicht. Diese Ausführungsform der Erfindung kann daher insbesondere für die Beschaffung der o. g. Gewebemodelle für die Wirkstoffprüfung Vorteile besitzen.

Gemäß einer ersten Variante besteht das Manipulationswerkzeug zumindest teilweise aus einem elastischen Material, das die Werkzeugoberfläche, die die Zellen berührt, bildet und das bei der Herstellung des Manipulationswerkzeugs gedehnt wird. Vorteilhafterweise bildet das elastische Material (z. B. Plastik, Gummi) eine geschlossene, stufenlose Oberfläche. Alternativ kann gemäß einer zweiten Variante die verstellbare Oberfläche aus einem nicht-elastischen Material bestehen, wie z. B. aus ineinander verschiebbaren Lamellen aus Metall oder Kunststoff, wobei sich Vorteile für die Genauigkeit der Veränderung der geometrischen Oberflächeneigenschaften ergeben können.

Wenn das Manipulationswerkzeug gemäß einer weiteren Modifikation der Erfindung einen Hohlkörper aufweist, der einen inneren Hohlraum mit einer inneren Oberfläche besitzt, die bei der Verstellung des Manipulationswerkzeugs vergrößert wird, können sich Vorteile für die Schaffung sphäroidförmiger Gewebemodelle ergeben. Gemäß verschiedenen Varianten der Erfindung kann das Zellmaterial außen auf dem hohlen Werkzeugkörper, auf der inneren Oberfläche des Werkzeugkörpers oder auf beiden inneren und äußeren Oberflächen angeordnet werden. Diese Varianten besitzen Vorteile für die Schaffung schichtförmiger Gewebemodelle aus verschiedenen Zellarten, die von außen oder innen auf die Werkzeugoberfläche aufwachsen können, wobei durch die erfindungsgemäße Verstellung der Oberfläche die zur Verfügung stehende Substratfläche laufend vergrößert wird. Eine unerwünscht hohe Schichtdicke, die zu einer Unterversorgung tiefer liegender Zellen führen könnte, kann vorteilhafterweise vermieden werden, wobei gleichzeitig erheblich größere Sphäroide als bei herkömmlichen Gewebemodellen gebildet werden können.

Das hohle Manipulationswerkzeug wird vorzugsweise kugelförmig mit einem Durchmesser im Bereich von 0.01 mm bis 10 cm gebildet.

Wenn die Bildung des Zellmaterials gemäß einer weiteren Variante des erfindungsgemäßen Verfahrens die geometrische Umordnung der Zellen durch die Verstellung der Werkzeugoberfläche und gleichzeitig ein Aufwachsen von Zellen aus einem Umgebungsmedium umfasst, können sich Vorteile für die Erzeugung von Zellkulturen mit einer vorgebbaren Maximaldicke ergeben. Ein unabhängiger Gegenstand der Erfindung ist entsprechend ein Zellkultivierungsverfahren, bei dem Zellen aus einem Kultivierungsmedium auf eine sich vergrößernde Substratoberfläche aufwachsen. Besondere Vorteile für die Gewebegestaltung (Tissue Engineering) können sich ergeben, wenn das Zellmaterial auf oder im hohlen Werkzeugkörper schichtweise aus Zellen verschiedener Zellarten gebildet wird.

Die Vergrößerung der Werkzeugoberfläche kann durch die Ausübung eines hydrostatischen Druckes auf das Innere des Manipulationswerkzeugs erfolgen. Beispielsweise kann gemäß einer vorteilhaften Variante der Erfindung das hohle Werkzeug aus einem elastischen Material mit einer Druckleitung (zum Beispiel einer Flüssigkeitsleitung) verbunden sein, durch die eine Flüssigkeit oder ein Gas in den Innenraum des Werkzeugs unter Druck einführbar ist. Die Dehnung der Werkzeugoberfläche erfolgt durch die Flüssigkeits- oder Gaszufuhr. Die Dehnung mit einer Flüssigkeit besitzt den Vorteil, dass das zugeführte Flüssigkeitsvolumen in direktem Zusammenhang mit der Vergrößerung der Oberfläche steht. Für die o. g. Deposition von Zellmaterial auf der Innenseite des Hohlkörpers kann es von Vorteil sein, wenn als Flüssigkeit zur Dehnung der Werkzeugoberfläche eine Zellsuspension verwendet wird, die die zu kultivierenden Zellen in den Innenraum befördert.

Für die Anwendung beim Tissue Engineering kann es vorteilhaft sein, wenn die Oberfläche des Werkzeugkörpers aus einem Material besteht, das einen elektrophysiologischen Kontakt, einen Stoffaustausch zwischen dem Innenraum des Werkzeugs und seiner Umgebung und/oder Zellkontakte zwischen dem Innen- und Außenraum erlaubt.

Gemäß einer weiteren, bevorzugten Ausführungsform der Erfindung enthält der Werkzeugkörper mindestens ein verschiebbares Formelement, wobei bei der Verstellung des Werkzeugkörpers das Formelement aus der Werkzeugoberfläche hervortritt und damit die Oberflächengestalt ändert. Das mindestens eine Formelement verleiht dem Werkzeugkörper die Funktion eines Stempels, dessen Stempelform durch die Verschiebbarkeit des Formelements variabel an die jeweiligen Anforderungen angepasst werden kann. Wenn eine Vielzahl von Formelementen vorgesehen ist, wird die Variabilität bei der Gestaltung der Stempeloberfläche vorteilhafterweise erhöht.

Gemäß einer Variante der Erfindung kann das Werkzeug mit dem mindestens einen Formelement als Zellkulturträger verwendet werden, der die Form der Zellkultur bestimmt. Alternativ kann das Manipulationswerkzeug mit der Oberfläche, deren Form durch die Anordnung des mindestens einen Formelements bestimmt ist, wie ein Stempel auf das Zellmaterial geschoben werden.

Ein unabhängiger Gegenstand der Erfindung ist ein mit dem erfindungsgemäßen Verfahren gebildetes biologisches Zellmaterial oder Zell-Träger-Verbundmaterial, wie zum Beispiel ein Zellsphäroid, der durch ein Zellmaterial auf einer gedehnten Trägeroberfläche gebildet wird.

Vorrichtungsbezogen wird die o. g. Aufgabe durch die Bereitstellung eines Manipulationswerkzeugs gelöst, das einen Werkzeugkörper mit mindestens einer Oberfläche, deren Form und/oder Größe verstellbar ist, und eine Stelleinrichtung zur Verstellung der Oberfläche umfasst. Vorteilhafterweise ermöglicht dieses Werkzeug bei Kontaktierung mit einem zu manipulierenden Zellmaterial, das dem Zellmaterial die Oberflächengestalt aufgeprägt wird. Wenn die Stelleinrichtung zur Veränderung der Oberflächengeometrie mit einer charakteristischen Vortriebsgeschwindigkeit entsprechend einer physiologischen Bezugsgeschwindigkeit biologischer Zellen eingerichtet ist, ergeben sich die o. g. Vorteile für eine verletzungsfreie Umordnung der Zellen bei der Formgebung.

Gemäß einer besonders vorteilhaften.Variante ist die Oberfläche des Manipulationswerkzeugs mit einer Struktur oder Beschichtung ausgestattet, die ein adhärentes Anhaften von biologischen Zellen fördert. Das Bindungsmaterial bildet den Werkzeugkörper, mindestens die Vorderseite des Werkzeugkörpers oder eine Beschichtung mindestens auf der Vorderseite des Werkzeugkörpers. Es besteht bspw. aus Fibronektin oder Kollagen. Diese Ausführungsform der Erfindung kann Vorteile in Bezug auf eine Erhöhung der Bindungsgeschwindigkeit bei der verdrängenden Bewegung des Werkzeugkörpers im Zellmaterial besitzen. Das Bindungsmaterial kann alternativ durch eine Aufrauhung mit charakteristischen Strukturgrößen im Sub-pm-Bereich besitzen, so dass die Anbindung des Zellmaterials an die Sonde gefördert wird.

Die Oberfläche des Manipulationswerkzeugs kann ferner mit einer Struktur oder Beschichtung ausgestattet sein, die ein adhärentes Anhaften von biologischen Zellen zumindest in Teilbereichen der Oberfläche blockiert. Als Beschichtung sind beispielsweise an sich bekannte chemische Verbindungen, zum Beispiel hydrophobe Silane und hydrophile Polymere vorgesehen. Weitere Einzelheiten und Vorteile der Erfindung werden im Folgenden unter Bezug auf die beigefügten Zeichnungen beschrieben. Es zeigen:
- Figuren 1 und 2:: Illustrationen der Bildung und Umordnung von Zellmaterial gemäß einer ersten Ausführungsform der Erfindung,
- Figur 3:: eine Illustration einer radialen Aufweitungsbewegung eines erfindungsgemäßen Manipulationswerkzeugs,
- Figuren 4 und 5:: eine Illustration einer Werkzeugoberfläche mit einer Vielzahl von verschiebbaren Formelementen, und
- Figur 6:: eine erfindungsgemäße Formprägung von Zellmaterial mit einem Manipulationswerkzeug.

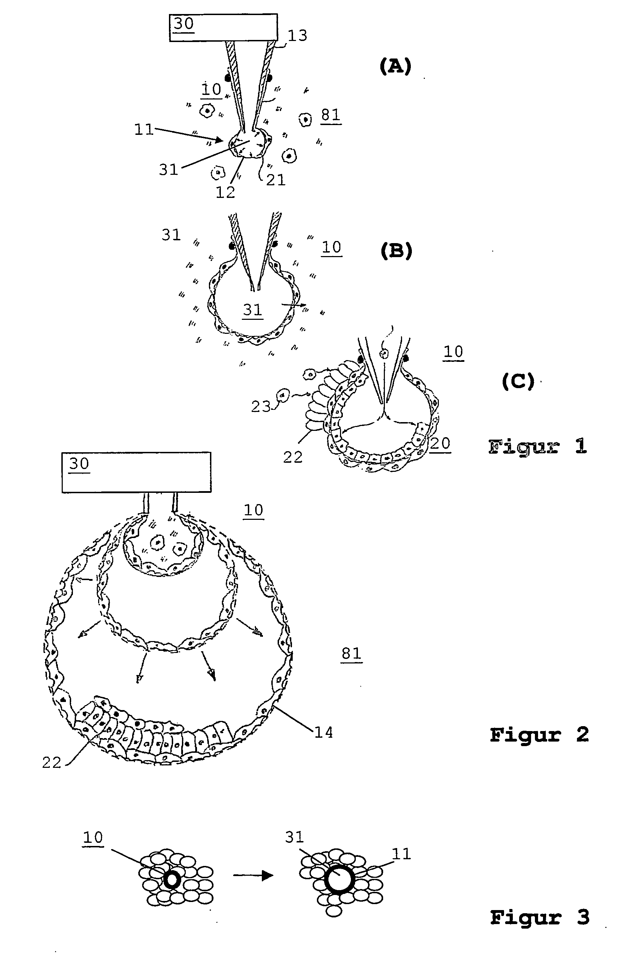

Die Figuren 1 und 2 illustrieren das erfindungsgemäße Prinzip, in vorbestimmter Weise Hohlkugeln oder andere Hohlgeometrien aus Zellmaterial zu erzeugen. Hierzu wird ein Manipulationswerkzeug 10 mit einem elastischen, hohlen Werkzeugkörper 11 und einer Druckleitung 13, über die eine externe Druckquelle 30 (nur in Teilbild A dargestellt) mit dem Innenraum 31 des Werkzeugkörpers 11 verbunden ist. Die schematisch gezeigte Druckquelle 30 bildet eine Stelleinrichtung zur Verstellung der Oberfläche 12 des Werkzeugkörpers 11. Sie umfasst zum Beispiel ein Flüssigkeitsreservoir und eine Pumpe.

Der Werkzeugkörper 11 bildet bspw. einen Ballon aus Gummi, der im entspannten Zustand einen Durchmesser von z. B. 0.01 mm besitzt und im gedehnten Zustand einen Durchmesser von bis zu z. B. 100 mm besitzen kann. Als Druckleitung 13 wird eine Kapillare z. B. aus Stahl oder Glas verwendet. Wenn das Material des Werkzeugs 10 aus teildurchlässigem, elastischen Kunststoffmaterial (z. B. Silikonmembran) besteht, wird vorteilhafterweise die Erzeugung von Stoffgradienten im Zellgewebe ermöglicht.

Durch das Eindrücken von Luft oder einer Flüssigkeit in den Innenraum 31 kann die Oberfläche 12 des Werkzeugskörpers 11 vergrößert werden, wobei die Form im wesentlichen erhalten bleibt. Die Vergrößerung erfolgt derart, dass sich benachbarte Bezugspunkte auf der Oberfläche 12 mit einer Geschwindigkeit entsprechend der o. g. physiologischen Bezugsgeschwindigkeit voneinander entfernen. Entsprechend können Zellen auf der Oberfläche 12 der Dehnbewegung folgen, ohne einer unerwünschten mechanischen Zerstörung ausgesetzt zu sein. Die Teilbilder A und B der Figur 1 zeigen, wie zunächst auf dem entspannten Werkzeugkörper eine Monolage von Zellen 21 aus einer umgebenden Zellsuspension 81 auf die Oberfläche 12 aufwächst. Durch eine Vergrößerung der Oberfläche 12 wird der Platz zur Vergrößerung der Zellmonolage 20 ständig vergrößert.

Durch die Zugabe weiterer Zelltypen 22, 23 in das Außenmedium und/oder das Innenmedium können flexibel gewebeartige Zellschichten erzeugt werden, wie dies schematisch im Teilbild C illustriert ist.

Figur 2 zeigt die Kultivierung von Zellmaterial 20 auf der inneren Oberfläche 14 des Hohlkörpers 11 in drei verschiedenen Dehnungszuständen. Vom Flüssigkeitsreservoir der Druckquelle 30 wird der Hohlraum 31 über die Druckleitung 13 mit einer Zellsuspension gefüllt. Adhärent wachsende Zellen (z. B. Fibroblasten, Makrophagen oder Tumorzellen) besiedeln die innere Oberfläche 14 aktiv. Bei vielen Zellarten setzt eine Kontaktinhibierung bei vollständiger Bedeckung der Substratoberfläche ein. Das heißt, eine weitere Vermehrung wird beendet, sobald eine Monolage gebildet ist. Durch die erfindungsgemäße Dehnung der Werkzeugoberfläche kann dieser Vorgang gezielt gesteuert werden.

Der Innenraum 31 kann, wie dies bei der größten Dehnungsvariante in Figur 2 gezeigt ist, mit verschiedenen Zellarten nacheinander oder gleichzeitig beschickt werden, so dass ein gewebeähnliches Zellmaterial mit einer Funktion entsprechend den an der Bildung des Zellmaterials beteiligten Zellarten erzeugt wird. Auch bei dieser Variante kann der Innenraum 31 in elektrophysiologischem Kontakt mit dem Außenmedium 81 stehen.

Figur 3 zeigt ein Beispiel einer radialen Aufweitungsbewegung eines kapillarförmigen Werkzeugs 10, dessen Körper 11 aus einem elastischen Material (zum Beispiel Plastik, Gummi) oder einem nicht-elastisch aufweitbaren Material (zum Beispiel relativ zueinander verschiebbare Lamellen aus Stahl oder Kunststoff o. dgl.) besteht. Durch eine Druckerhöhung im Hohlkanal 31 des Werkzeugs 10 kann dessen Durchmesser erweitert werden, wobei eine verletzungsfreie Verdrängung der Zellen erfolgt. Der Durchmesser des Werkzeugkörpers 11 vergrößert sich mit der oben beschriebenen Vortriebsgeschwindigkeit.

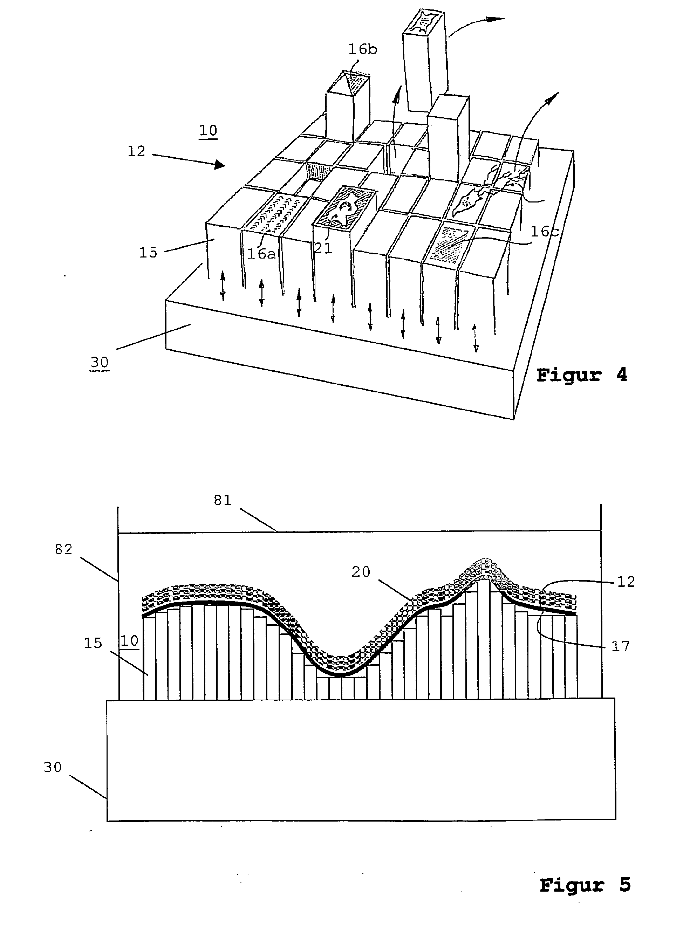

Die Figuren 4 und 5 illustrieren ein Manipulationswerkzeug 10, das eine Vielzahl von Formelementen 15 aufweist, die jeweils einzeln mit einer Stelleinrichtung 30 verschiebbar sind, die an einem Basisteil (nicht dargestellt) angebracht ist. Jedes Formelement 15 besitzt eine Quaderform mit einer Oberseite 16. Die Gesamtzahl der Oberseiten 16 oder mindestens ein auf diesen angeordnetes, nachgiebiges, schichtförmiges Abdeckelement 17 (siehe Figur 5) bilden die Oberfläche 12 des Manipulationswerkzeugs 10, die zur Formgebung an Zellmaterial 20 verwendet wird. Die Formelemente 15 bilden den Werkzeugkörper. Die Oberseiten 16 besitzen typische Dimensionen im Bereich von 0.01 mm bis 5 mm. Die Formelemente 15 sind so ausgerichtet, dass die Oberseiten 16 eine Matrixanordnung aus geraden Reihen und Spalten bilden. Die Verschiebung der Formelemente 15 relativ zum Basisteil erfolgt bspw. mit Stellmotoren oder piezoelektrischen Antrieben. Je nach dem gewählten Vorschub eines Formelements 15 wird die Werkzeugoberfläche strukturiert. Einzelne Formelemente 15 können von der Stelleinrichtung 30 trennbar sein, zum Beispiel um Löcher im Zellmaterial zu bilden.

Das Abdeckelement 17 besitzt die Vorteile, dass die Werkzeugoberfläche lokal geglättet und die Abnahme des Zellmaterials vom Werkzeug erleichtert wird. Das Abdeckelement 17 umfasst zum Beispiel eine Folie (zum Beispiel Polyurethan) oder eine Membran, die sich über alle Formelemente 15 erstreckt und auf der die Zellen angeordnet sind. Es können alternativ ein oder mehrere Abdeckelemente 17 vorgesehen sein die sich nur über eine oder mehrere Teilgruppen von Formelementen 15 erstrecken. Es kann ein lösbares Haften des Abdeckelements 17 an einigen oder allen Oberseiten 16 der Formelemente 15 vorgesehen sein.

Das Abdeckelement 17 kann aus einem synthetischen Polymermaterial und/oder aus einem in biologischen Organismen natürlich vorkommenden Material, wie zum Beispiel Chitin oder Knochenmatrixmaterial, in einer oder mehreren Schichten bestehen. Das Abdeckelement 17 kann ferner eine strukturierte Beschichtung tragen, die ein adhärentes Anhaften von biologischen Zellen in Teilbereichen einerseits fördert und in anderen Teilbereichen andererseits blockiert.

Zur erfindungsgemäßen Bildung von Zellmaterial wird dieses zunächst auf der Werkzeugoberfläche 12, also der Gesamtheit der Oberseiten 16 oder dem gemeinsamen Abdeckelement 17 angeordnet. Hierzu ist bspw. ein Aufwachsen aus einem Kultivierungsmedium 81 in einem Kulturgefäß 82 (siehe Figur 5) vorgesehen. Aus Übersichtlichkeitsgründen sind in Figur 4 nur einzelne Zellen 21 gezeigt. Anschließend erfolgt eine Verstellung der Oberfläche des Manipulationswerkzeugs 10 durch den Vorschub der Formelemente 15 in die jeweils gewünschten Positionen. Dieser Vorschub erfolgt mit der o. g. physiologischen Bezugsgeschwindigkeit, so dass bei der Deformation des Zellmaterials eine verletzungsfreie Verdrängung und Umordnung der Zellen erfolgt. Anschließend kann eine Ablösung des Zellmaterials vom Manipulationswerkzeug 10 erfolgen.

Figur 4 illustriert auch, dass die einzelnen Oberseiten 16 der Formelemente 15 verschieden gebildet sein können, um an den jeweiligen Positionen das Zellmaterial zusätzlich zu modifizieren. Es können bspw. Mikrostrukturen (s. bei 16a) zur Verbesserung der Haftfähigkeit der Oberseite 16 oder zusätzliche Strukturelemente wie z. B. die Pyramidenform 16b zur Schaffung einer Struktur im Zellmaterial vorgesehen sein. Des Weiteren können einzelne oder alle Formelemente 15 eine adhäsionssteigernde Beschichtung 16c tragen.

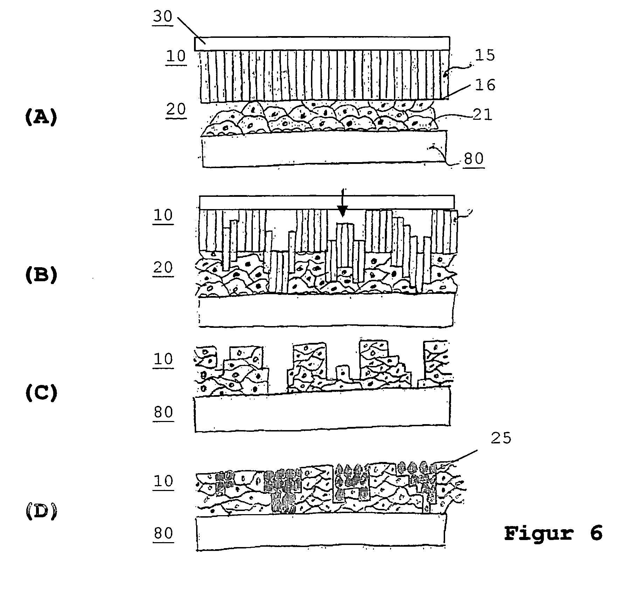

Ein Manipulationswerkzeug 10 gemäß Figur 4 kann alternativ als Abdruckwerkzeug verwendet werden, wie dies in der Bildfolge gemäß Figur 6 illustriert ist. In einer Ausgangssituation gemäß Teilbild A befindet sich auf einem Träger 80 ein Zellmaterial 20, das entsprechend dem erfindungsgemäßen Verfahren deformiert werden soll. Über der zunächst freien Oberfläche des Zellmaterials 20 wird das Manipulationswerkzeug 10 mit der Vielzahl von verschiebbaren Formelementen 15 angeordnet. Das Manipulationswerkzeug 10 wird an das Zellmaterial 20 angenähert, bis die in diesem Fall nach unten weisenden Oberseiten 16 das Zellmaterial 20 berühren. Anschließend erfolgt gemäß Teilbild B eine Verstellung der Oberfläche 11 des Manipulationswerkzeugs 10 durch den gezielten Vorschub einzelner Formelemente 15. Die Vorschubbewegung erfolgt mit der o. g. physiologischen Bezugsgeschwindigkeit biologischer Zellen. Die einzelnen Formelemente 15 verdrängen die Zellen im Zellmaterial verletzungsfrei.

Anschließend wird gemäß Teilbild C das Manipulationswerkzeug 10 entfernt. Die Oberflächenform des Werkzeugs bleibt als komplementäre Struktur im Zellmaterial 10 bestehen. Für eine Erleichterung der Abtrennung des Manipulationswerkzeugs 10 vom Zellmaterial 20 können die Oberseiten 16 der Formelemente 15 mit einer Beschichtung versehen sein, auf der ein Anhaften von Zellen unterbunden wird. Die Beschichtung erfolgt bspw. mit dem Polymer Polyhema. Schließlich können die im Zellmaterial eingeprägten Lücken gemäß Teilbild D mit anderen Zellen oder einem synthetischen Matrixmaterial 25 befüllt werden.

Die Auswahl der Form und ggf. in das Zellmaterial zugeführten Zellen oder Zusatzstoffe 25 erfolgt je nach der konkreten Aufgabe im Rahmen des Tissue Engineering. Mit der Abfolge gemäß Figur 6 können bspw. Epithel-Zellen mit einer vorbestimmten Struktur mit Gewebe-Zellen in Verbindung gebracht werden.

## Patentansprüche

1. Verfahren zur Bildung von Zellmaterial (20) mit einer Vielzahl biologischer Zellen (21), die eine vorbestimmte geometrische Anordnung aufweisen,
**gekennzeichnet durch** die Schritte:
- Bereitstellung eines Manipulationswerkzeugs (10) mit einem Werkzeugkörper (11), dessen Oberfläche (12, 14) das Zellmaterial (20) zumindest teilweise berührt, und
- Verstellung des Manipulationswerkzeugs (10) mit einer Veränderung von mindestens einem der Merkmale Fläche, Ausrichtung relativ zu einem ortsfesten Bezugssystem, Oberflächenform und Oberflächenstruktur der Oberfläche (12, 14) derart, dass die geometrische Anordnung der Zellen (21) verändert wird.

2. Verfahren nach Anspruch 1, bei der die Verstellung des Manipulationswerkzeugs (10) mit einer Vortriebsgeschwindigkeit erfolgt, die kleiner oder gleich einer physiologischen Bezugsgeschwindigkeit der Zellen ist.

3. Verfahren nach Anspruch 2, bei dem die Verstellung des Manipulationswerkzeugs (10) mit einer Vortriebsgeschwindigkeit im Bereich von 0.1 um/h bis 1 mm/h erfolgt.

4. Verfahren nach mindestens einem der vorgehenden Ansprüche, bei dem bei der Verstellung des Manipulationswerkzeugs (10) die Oberfläche (12, 14) des Manipulationswerkzeugs (10) vergrößert wird.

5. Verfahren nach Anspruch 4, bei dem das Manipulationswerkzeug (10) ein elastisches Material aufweist, das bei der Verstellung gedehnt wird.

6. Verfahren nach Anspruch 4, bei dem das Manipulationswerkzeug (10) ein nicht-elastisches Material aufweist, das bei der Verstellung aufgeweitet wird.

7. Verfahren nach Anspruch 5 oder 6, bei dem das Manipulationswerkzeug (10) einen Hohlkörper (11) mit einem Innenraum (31) aufweist, der bei der Verstellung vergrößert wird.

8. Verfahren nach Anspruch 7, bei dem das Zellmaterial außen auf dem Hohlkörper (11) und/oder im Innenraum (31) des Hohlkörpers (11) angeordnet ist.

9. Verfahren nach Anspruch 8, bei dem während der Verstellung der Oberfläche (12, 14) ein Anhaften von einzelnen Zellen oder Zellgruppen auf der Oberfläche (12, 14) außen auf und/oder im Hohlkörper (11) erfolgt.

10. Verfahren nach Anspruch 7 oder 8, bei dem das Zellmaterial außen auf und/oder im Hohlkörper (11) schichtweise aus verschiedenen Zellarten angeordnet wird.

11. Verfahren nach mindestens einem der vorgehenden Ansprüche 7 bis 10, bei dem zur Verstellung des Hohlkörpers (11) der Innenraum (31) über eine Druckleitung (13) mit einem Druck beaufschlagt wird.

12. Verfahren nach Anspruch 11, bei dem zur Verstellung des Hohlkörpers (11) eine Zellsuspension in den Innenraum eingeführt wird.

13. Verfahren nach mindestens einem der vorgehenden Ansprüche 7 bis 12; bei dem zwischen dem Innenraum des Hohlkörpers (11) und seiner Umgebung ein Stoffaustausch erfolgt.

14. Verfahren nach Anspruch 4, bei dem der Werkzeugkörper mindestens ein Formelement (15) enthält, das bei der Verstellung des Manipulationswerkzeugs (10) verschoben wird, so dass sich die Form der Oberfläche (12) verändert.

15. Verfahren nach Anspruch 14, bei dem der Werkzeugkörper eine Vielzahl von Formelementen (15) enthält, die bei der Verstellung des Manipulationswerkzeugs (10) verschoben werden.

16. Verfahren nach mindestens einem der vorgehenden Ansprüche, bei dem bei der Verstellung des Manipulationswerkzeugs (10) die Oberfläche (12) und/oder die Formelemente (15) zumindest teilweise in das Zellmaterial (20) geschoben werden und im Zellmaterial (20) eine Abdruckform der Oberfläche (12) des Manipulationswerkzeugs (10) gebildet wird.

17. Verfahren nach Anspruch 16, bei dem auf dem Zellmaterial (20) weitere Zellen oder ein synthetisches Matrixmaterial (25) angeordnet werden.

18. Verfahren nach mindestens einem der vorgehenden Ansprüche, bei dem das Zellmaterial (20) ein Verbundmaterial umfasst, das aus den biologischen Zellen oder aus den biologischen Zellen und einem Matrixmaterial besteht.

19. Verfahren nach mindestens einem der vorgehenden Ansprüche, bei dem die geometrische Anordnung der Zellen (21) mit dem Manipulationswerkzeug (10) außerhalb eines tierischen oder menschlichen Organismus verändert wird.

20. Manipulationswerkzeug (10) zur Bildung von Zellmaterial (20) mit einer Vielzahl biologischer Zellen (21), die eine vorbestimmte geometrische Anordnung aufweisen,
**gekennzeichnet durch:**
- einen Werkzeugkörper (11) mit mindestens einer Oberfläche (12, 14), und
- einer Stelleinrichtung (30) zur Verstellung des Werkzeugkörpers (11), so dass sich mindestens eines der Merkmale Fläche, Ausrichtung relativ zu einem ortsfesten Bezugssystem, Oberflächenform und Oberflächenstruktur der Oberfläche (12, 14) verändert.

21. Manipulationswerkzeug nach Anspruch 20, bei dem die Stelleinrichtung (30) zur Verstellung des Werkzeugkörpers (11) mit einer Vortriebsgeschwindigkeit eingerichtet ist, die kleiner oder gleich einer physiologischen Bezugsgeschwindigkeit der Zellen (21) ist.

22. Manipulationswerkzeug nach Anspruch 21, bei dem die Stelleinrichtung (30) zur Verstellung des Werkzeugkörpers (11) mit einer Vortriebsgeschwindigkeit im Bereich von 0.1 µm/h bis 1 mm/h eingerichtet ist.

23. Manipulationswerkzeug nach mindestens einem der Ansprüche 20 bis 22, bei dem die Oberfläche (12) des Werkzeugkörpers (11) dazu eingerichtet ist, bei der Verstellung vergrößert zu werden.

24. Manipulationswerkzeug nach Anspruch 23, bei dem der Werkzeugkörper (11) ein elastisches Material aufweist.

25. Manipulationswerkzeug nach Anspruch 23, bei dem der Werkzeugkörper (11) ein nicht-elastisches, aufweitbares Material aufweist.

26. Manipulationswerkzeug nach Anspruch 24 oder 25, bei dem der Werkzeugkörper ein Hohlkörper (11) mit einem Innenraum (31) umfasst, der bei der Verstellung vergrößert wird.

27. Manipulationswerkzeug nach Anspruch 26, bei dem der Hohlkörper (11) über eine Druckleitung (13) mit einer Druckquelle (30) verbunden ist.

28. Manipulationswerkzeug nach Anspruch 23, bei dem der Werkzeugkörper (11) mindestens ein verschiebbares Formelement (15) enthält.

29. Manipulationswerkzeug nach Anspruch 28, bei dem der Werkzeugkörper (11) eine Vielzahl von verschiebbaren Formelementen (15) enthält, die matrixförmig in geraden Reihen und Spalten angeordnet sind.

30. Manipulationswerkzeug nach Anspruch 28 oder 29, bei dem auf den Formelementen (15) mindestens ein Abdeckelement (17) angeordnet ist, das die Oberfläche (12) bildet.

31. Manipulationswerkzeug nach mindestens einem der Ansprüche 20 bis 30, bei dem auf der Oberfläche (12) eine Struktur oder Beschichtung vorgesehen ist, die ein adhärentes Anhaften von biologischen Zellen fördert.

32. Manipulationswerkzeug nach mindestens einem der Ansprüche 20 bis 31, bei dem auf der Oberfläche (12) eine Struktur oder Beschichtung vorgesehen ist, die ein adhärentes Anhaften von biologischen Zellen blockiert.

## Claims

1. A method for producing cell material (20) having multiple biological cells (21), which have a predefined geometrical arrangement,
**characterized by** the steps:
- providing a manipulation tool (10) having a tool body (11), whose surface (12, 14) at least partially contacts the cell material (20), and
- adjusting the manipulation tool (10) including a change of at least one of the features of area, orientation in relation to a fixed reference system, surface shape and surface structure of the surface (12, 14) in such way that the geometrical arrangement of the cells (21) is changed.

2. The method according to Claim 1, wherein the adjustment of the manipulation tool (10) is performed at an advance velocity which is less than or equal to a physiological reference velocity of the cells.

3. The method according to Claim 2, wherein the adjustment of the manipulation tool (10) is performed at an advance velocity in the range from 0.1 um/h to 1 mm/h.

4. The method according to at least one of the preceding claims, wherein, during the adjustment of the manipulation tool (10), the surface (12, 14) of the manipulation tool (10) is enlarged.

5. The method according to Claim 4, wherein the manipulation tool (10) has an elastic material that is stretched during the adjustment.

6. The method according to Claim 4, wherein the manipulation tool (10) has a non-elastic material that is expanded during the adjustment.

7. The method according to Claim 5 or 6, wherein the manipulation tool (10) has a hollow body (11) having an interior (31) which is enlarged during the adjustment.

8. The method according to Claim 7, wherein the cell material is positioned externally on the hollow body (11) and/or in the interior (31) of the hollow body (11).

9. The method according to Claim 8, wherein, during the adjustment of the surface (12, 14), individual cells or cell groups adhere externally on the surface (12, 14) and/or in the hollow body (11).

10. The method according to Claim 7 or 8, wherein the cell material is positioned in layers made of different types of cells externally on and/or in the hollow body (11).

11. The method according to at least one of the preceding Claims 7 through 10, wherein the interior (31) has a pressure applied to it via a pressure line (13) to adjust the hollow body (11).

12. The method according to Claim 11, wherein a cell suspension is introduced into the interior to adjust the hollow body (11).

13. The method according to at least one of the preceding Claims 7 through 12, wherein a mass transfer occurs between the interior of the hollow body (11) and its environment.

14. The method according to Claim 4, wherein the tool body includes at least one shaping element (15), which is displaced during the adjustment of the manipulation tool (10), so that the shape of the surface (12) changes.

15. The method according to Claim 14, wherein the tool body contains multiple shaping elements (15), which are displaced during the adjustment of the manipulation tool (10).

16. The method according to at least one of the preceding claims, wherein, during the adjustment of the manipulation tool (10), the surface (12) and/or the shaping elements (15) are at least partially pushed into the cell material (20) and an imprint shape of the surface (12) of the manipulation tool (10) is formed in the cell material (20).

17. The method according to Claim 16, wherein further cells or a synthetic matrix material (25) are positioned on the cell material (20).

18. The method according to at least one of the preceding claims, wherein the cell material (20) comprises a composite material that is made of the biological cells or of the biological cells and a matrix material.

19. The method according to at least one of the preceding claims, wherein the geometrical arrangement of the cells (21) is changed using the manipulation tool (10) outside an animal or human organism.

20. A manipulation tool (10) for producing cell material (20) having multiple biological cells (21), which have a predefined geometrical arrangement,
**characterized by:**
- a tool body (11) having at least one surface (12, 14), and
- a setting device (30) for adjusting the tool body (11), so that at least one of the features of area, orientation relative to a fixed reference system, surface shape and surface structure of the surface (12, 14) change.

21. The manipulation tool according to Claim 20, wherein the setting device (30) is set up for adjusting the tool body (11) at an advance velocity which is less than or equal to a physiological reference velocity of the cells (21).

22. The manipulation tool according to Claim 21, wherein the setting device (30) is set up for adjusting the tool body (11) at an advance velocity in the range from 0.1 um/h to 1 mm/h.

23. The manipulation tool according to at least one of Claims 20 through 22, wherein the surface (12) of the tool body (11) is set up for the purpose of being enlarged during the adjustment.

24. The manipulation tool according to Claim 23,
wherein the tool body (11) has an elastic material.

25. The manipulation tool according to Claim 23, wherein the tool body (11) has a non-elastic, expandable material.

26. The manipulation tool according to Claim 24 or 25, wherein the tool body comprises a hollow body (11) having an interior (31) which is enlarged during the adjustment.

27. The manipulation tool according to Claim 26, wherein the hollow body (11) is connected to a pressure source (30) via a pressure line (13).

28. The manipulation tool according to Claim 23, wherein the tool body (11) contains at least one displaceable shaping element (15).

29. The manipulation tool according to Claim 28, wherein the tool body (11) contains multiple displaceable shaping elements (15), which are positioned in linear rows and columns in a matrix.

30. The manipulation tool according to Claim 28 or 29, wherein at least one cover element (17), which forms the surface (12), is positioned on the shaping elements (15).

31. The manipulation tool according to at least one of Claims 20 through 30, wherein a structure or coating, which encourages adherent adhesion of biological cells, is provided on the surface (12).

32. The manipulation tool according to at least one of Claims 20 through 31, wherein a structure or coating which blocks adherent adhesion of biological cells is provided on the surface (12).

## Revendications

1. Procédé de formation d'une matière cellulaire (20) avec une multitude de cellules biologiques (21) qui présentent une disposition géométrique prédéterminée, **caractérisé par** les étapes consistant à :
- mettre à disposition un outil de manipulation (10) avec un corps d'outil (11), dont la surface (12, 14) touche au moins partiellement la matière cellulaire (20), et
- régler l'outil de manipulation (10) en modifiant au moins une des caractéristiques parmi surface, orientation par rapport à un système de référence fixe, forme superficielle et structure superficielle de la surface (12, 14), de telle façon que la disposition géométrique des cellules (21) soit modifiée.

2. Procédé selon la revendication 1, dans lequel le réglage de l'outil de manipulation (10) s'effectue avec une vitesse d'avancement qui est inférieure ou égale à la vitesse de référence physiologique des cellules.

3. Procédé selon la revendication 2, dans lequel le réglage de l'outil de manipulation (10) s'effectue avec une vitesse d'avancement située dans la plage de 0,1 um/h à 1 mm/h .

4. Procédé selon au moins une des revendications précédentes, dans lequel la surface (12, 14) de l'outil de manipulation (10) est augmentée lors du réglage de l'outil de manipulation (10).

5. Procédé selon la revendication 4, dans lequel l'outil de manipulation (10) présente une matière élastique qui est étirée lors du réglage.

6. Procédé selon la revendication 4, dans lequel l'outil de manipulation (10) présente une matière non élastique qui est élargie radialement lors du réglage.

7. Procédé selon la revendication 5 ou 6, dans lequel l'outil de manipulation (10) présente un corps creux (11) doté d'un espace intérieur (31), qui est agrandi lors du réglage.

8. Procédé selon la revendication 7, dans lequel la matière cellulaire est disposée à l'extérieur sur le corps creux (11) et/ou dans l'espace intérieur (31) du corps creux (11).

9. Procédé selon la revendication 8, dans lequel il se produit, pendant le réglage de la surface (12, 14), un ancrage des différentes cellules ou groupes de cellules sur la surface (12, 14) à l'extérieur et/ou à l'intérieur du corps creux (11).

10. Procédé selon la revendication 7 ou 8, dans lequel la matière cellulaire est disposée à l'extérieur sur le corps creux (11) et/ou dans le corps creux (11) en couches de différentes espèces cellulaires.

11. Procédé selon au moins une des revendications précédentes 7 à 10, dans lequel l'espace intérieur (31) est soumis à une pression par le biais d'une conduite de pression (13) pour le réglage du corps creux (11).

12. Procédé selon la revendication 11, dans lequel une suspension cellulaire est introduite dans l'espace intérieur pour le réglage du corps creux (11).

13. Procédé selon au moins une des revendications précédentes 7 à 12, dans lequel un échange de substances s'effectue entre l'espace intérieur du corps creux (11) et son environnement.

14. Procédé selon la revendication 4, dans lequel le corps d'outil comprend au moins un élément de forme (15) qui coulisse lors du réglage de l'outil de manipulation (10) si bien que la forme de la surface (12) se modifie.

15. Procédé selon la revendication 14, dans lequel le corps d'outil comprend une multitude d'éléments de forme (15) qui coulissent lors du réglage de l'outil de manipulation (10).

16. Procédé selon au moins une des revendications précédentes, dans lequel la surface (12) et/ou les éléments de forme (15) sont poussés au moins partiellement dans la matière cellulaire (20) lors du réglage de l'outil de manipulation (10) et une empreinte de la surface (12) de l'outil de manipulation (10) est formée dans la matière cellulaire (20).

17. Procédé selon la revendication 16, dans lequel d'autres cellules ou une matière synthétique de matrice (25) sont disposées sur la matière cellulaire (20).

18. Procédé selon au moins une des revendications précédentes, dans lequel la matière cellulaire (20) comprend un matière composite constituée de cellules biologiques ou de cellules biologiques et d'une matière de matrice.

19. Procédé selon au moins une des revendications précédentes, dans lequel la disposition géométrique des cellules (21) est modifiée avec l'outil de manipulation (10) à l'extérieur d'un organisme animal ou humain.

20. Outil de manipulation (10) pour la formation d'une matière cellulaire (20) comprenant une multitude de cellules biologiques (21) qui présentent une disposition géométrique prédéterminée, **caractérisé par** :
- un corps d'outil (11) doté d'au moins une surface (12, 14), et
- un système de réglage (30) pour le réglage du corps d'outil (11) permettant de modifier au moins une des caractéristiques parmi surface, orientation par rapport à un système de référence fixe, forme superficielle et structure superficielle de la surface (12, 14).

21. Outil de manipulation selon la revendication 20, dans lequel le système de réglage (30) pour le réglage du corps d'outil (11) est pourvu d'une vitesse d'avancement qui est inférieure ou égale à la vitesse de référence physiologique des cellules (21).

22. Outil de manipulation selon la revendication 21, dans lequel le système de réglage (30) pour le réglage du corps d'outil (11) est doté d'une vitesse d'avancement située dans la plage de 0,1 µm/h à 1 mm/h.

23. Outil de manipulation selon au moins une des revendications 20 à 22, dans lequel la surface (12) du corps d'outil (11) est conçue pour être agrandie lors du réglage.

24. Outil de manipulation selon la revendication 23, dans lequel le corps d'outil (11) présente une matière élastique.

25. Outil de manipulation selon la revendication 23, dans lequel le corps d'outil (11) présente une matière non élastique extensible.

26. Outil de manipulation selon la revendication 24 ou 25, dans lequel le corps d'outil présente un corps creux (11) doté d'un espace intérieur (31), qui est agrandi lors du réglage.

27. Outil de manipulation selon la revendication 26, dans lequel le corps creux (11) est relié à une source de pression (30) par le biais d'une conduite de pression (13).

28. Outil de manipulation selon la revendication 23, dans lequel le corps d'outil (11) comprend au moins un élément de forme (15) coulissant.

29. Outil de manipulation selon la revendication 28, dans lequel le corps d'outil (11) comporte une multitude d'éléments de forme (15) coulissants, qui sont disposés en forme de matrice en lignes droites et en colonnes.

30. Outil de manipulation selon la revendication 28 ou 29, dans lequel au moins un élément de recouvrement (17) formant la surface (12) est disposé sur les éléments de forme (15).

31. Outil de manipulation selon au moins une des revendications 20 à 30, dans lequel une structure ou un revêtement, qui favorise un ancrage adhérent des cellules biologiques, est prévu(e) sur la surface (12).

32. Outil de manipulation selon au moins une des revendications 20 à 31, dans lequel une structure ou un revêtement, qui empêche un ancrage adhérent des cellules biologiques, est prévu(e) sur la surface (12).
